# EUROPEAN PATENT SPECIFICATION

(11) **EP 4 003 434 B1**
(45) Date of publication and mention of the grant of the patent: **20.12.2023**
(21) Application number: 20761330.8
(22) Date of filing: 14.07.2020
(51) Int. Cl.: A61L 2/18, A61B 90/70, A61L 2/26, B08B 3/06

(54) **POSITIONING AND SUPPORT DEVICE FOR ENDOSCOPIC INSTRUMENTS TO BE SUBJECTED TO WASHING**
POSITIONIERUNGS- UND STÜTZVORRICHTUNG FÜR ENDOSKOPISCHE, ZU WASCHENDE INSTRUMENTE
DISPOSITIF DE POSITIONNEMENT ET DE SUPPORT POUR INSTRUMENTS ENDOSCOPIQUES À SOUMETTRE À UN LAVAGE

(30) Priority: 29.07.2019 IT 201900013233
(43) Date of publication of application: 01.06.2022
(73) Proprietor: Steelco S.p.A., 31039 Riese Pio X (IT)
(72) Inventor: CALZAVARA, Francesco, 31033 CASTELFRANCO VENETO (IT)
(74) Representative: Petraz, Gilberto Luigi
(86) International application number: PCT/IT2020/050173
(87) International publication number: WO 2021/019584

(56) References cited:
- EP-A1- 1 949 868
- WO-A1-96/41686
- Valerio Poggi: "Riparazione lavatrice fai da te", , 13 March 2017 (2017-03-13), XP055678853, Retrieved from the Internet: URL:https://www.lavorincasa.it/riparazione -lavatrice-fai-da-te/ [retrieved on 2020-03-23]

## Description

### FIELD OF THE INVENTION

Embodiments described here concern a positioning and support device that can be used to perform targeted washing treatments on a distal end of endoscopic instruments, that is, the terminal part of the endoscopic instrument that is normally introduced inside a human body. Said positioning and support device can be used in itself, or be used in cooperation with a washing rack or tray, and possibly integrated as part of a rack or tray.

Embodiments described here also concern a method for treating an endoscopic instrument, for example washing, disinfection or sterilization.

### BACKGROUND OF THE INVENTION

It is known that flexible endoscopes are commonly used for both diagnostic and also for therapeutic purposes. For example, endoscopes can be used to diagnose and/or treat medical pathologies involving the lungs, esophagus, stomach, small intestine, biliary tract, pancreas or colon.

It is also known that endoscopes are instruments that are reused numerous times on different patients and therefore a high-level disinfection is necessary in order to eliminate any bacterial loads that could contaminate a subsequent patient.

In the absence of adequate disinfection, in fact, in patients undergoing endoscopy, infections can occur which can be the result of the flora of the previous patient, or the result of microbes introduced through the endoscope.

Endoscopes generally consist of one or more materials that can be chemically sensitive or that can degrade over time and with use.

In addition, due to the complex shape and the components and adhesives used that are sensitive to heat, endoscopes cannot be sterilized in an autoclave by steam.

Consequently, most modern endoscopes cannot be sterilized, indicating by this term the complete removal or destruction of all cells, or microbial spores that may be present on them.

Endoscopes are therefore subjected to disinfection, meaning by this term the removal or elimination of most, but not all, microorganisms.

Many treatment systems use a combination of chemical agents and moderate heat to obtain a high level of disinfection, but these may not be sufficient, for example in the treatment of duodenoscopes which have a complex structure.

Duodenoscopes, in fact, have a specific structure in correspondence with their distal end, which can be used to modify the angle of the catheters, called the elevator, or fin, and a channel associated with it.

The elevator can be rotated between a lowered position, in which it is substantially aligned with a longitudinal axis of the endoscope, to allow it to slide inside the human body, and a raised position, in which it is rotated with respect to said axis and protrudes in the transverse direction to be able to exert an opening action on a part of the duodenum.

This constructive complexity makes disinfection more difficult, and this greater difficulty contributes to a high risk of infection when compared to the risk associated with other types of endoscopes (for example gastroscopes).

In recent years, several cases have occurred of patients exposed to multi-drug resistant organisms (MDRO), for example Carbapenem-resistant enterobacteria (CRE) due to contaminated duedonoscopes.

The cases of transmission of CRE have been associated with the use of contaminated duodenoscopes used to perform endoscopic retrograde cholangiopancreatography (ERCP), a procedure that is currently performed even half a million times every year.

However, CRE are only the infectious agents that have recently been associated with endoscopes that have turned out to be contaminated, but many other pathologies that can be transmitted through endoscopic procedures are also known.

To try to solve the problem, regulations and guidelines have been defined that provide a specific washing and disinfection treatment for duodenoscopes, and in particular for their distal end which is provided with the elevator.

Because of the elaborate and non-linear shape of the distal end, due to the presence of an elevator, bacteria and microorganisms can nest in the interstices that are formed which, if not properly removed, can be extremely harmful for patients.

In particular, the international guidelines commonly adopted for the sanitization of duodenoscopes, expressly require that the distal end in question must be subjected to specific, dedicated and localized treatment, in order to remedy the critical issues expressed above.

The guidelines require manual pre-washing, using brushes, or pipe cleaners, of the distal end, and subsequent washing using suitable treatment machines.

For example, treatment machines are known which provide to treat the endoscopic instrument by immersion. These immersion treatment machines, however, require the use of large quantities of treatment liquid and in any case do not guarantee an optimal level of disinfection in the case of treating the distal end of a duodenoscope.

Apparatuses for washing endoscopes are also known, provided with support devices, and in which the articles to be washed and disinfected are positioned and clamped in an adequate manner. The articles are then hit by jets of washing liquid in suitable treatment machines. Even these known solutions, however, do not guarantee specific cleaning of the distal end of the duodenoscopes, since the protruding elevator tends to form shadow zones which hinder the jets and the splashes of treatment fluid and in fact invalidate the effectiveness of the cleaning treatment.

Document EP-A-1 949 868 describes a washing device for a medical instrument of the tubular type, comprising a first external tubular element, means to deliver a washing liquid in the first tubular element, and a second tubular element disposed inside the first tubular element, defining with it an interspace and provided with a plurality of slits through which the washing liquid can pass. In the washing device described here, the entry of the washing liquid and the discharge outlet are located in opposite positions to each other in relation to the longitudinal extension of the device, and the water is introduced into the interspace in a radial direction. This conformation does not allow to obtain a uniform distribution of the washing liquid in the interspace and toward the medical instrument, making this solution in fact unsuitable for washing the distal end of a duodenoscope, which has all the problems described above. Furthermore, since the interspace is closed in the direction of the discharge aperture, the washing fluid can accumulate in it.

Document WO-A-96/41686 describes a device for treating and disinfecting medical instruments comprising a treatment chamber having an external wall and an internal wall, the latter provided with a plurality of holes, separated by an interspace divided in an axial direction into truncated cone spaces. The interspace is connected to a sleeve provided with a plurality of valves to introduce a mixture of air and water in a radial direction into each sector; the mixture, through the holes, reaches the instrument to be washed and is finally sucked up and discharged through an exit aperture by the Venturi effect. This solution, which requires to deliver jets of fluid for each sector, entails a complex construction, and electronic elements such as electro valves, and therefore it is not suitable to be inserted inside a treatment and washing machine.

There is therefore a need to perfect a positioning and support device that can be used for the washing, sterilization and/or thermo-disinfection treatment of the distal end of an endoscopic instrument, for example, in the case of a duodenoscope, where the elevator is present, which can overcome at least one of the disadvantages of the state of the art.

In particular, one purpose of the present invention is to provide a positioning and support device which allows great simplicity of use.

Another purpose of the present invention is to provide a positioning and support device for washing the distal end of an endoscope which can be used both for itself and in any treatment machine.

Another purpose of the present invention is to provide a positioning and support device which allows to increase the efficiency of the washing treatment of the distal end of endoscopes, and in particular of duodenoscopes.

Another purpose is to provide a positioning and support device which is also simple to clean and sterilize.

One purpose of the present invention is also to provide a positioning and support device which facilitates the discharge of the washing fluid, preventing any accumulation thereof inside it.

Another purpose of the invention is to provide a positioning and support device that can be easily adapted to different configurations of use, alone or in combination with known racks or trays for treating endoscopic instruments.

Another purpose of the present invention is to perfect a method for treating endoscopes, in particular duodenoscopes, which allows to perform an accurate treatment of the distal end thereof.

The Applicant has devised, tested and embodied the present invention to overcome the shortcomings of the state of the art and to obtain these and other purposes and advantages.

### SUMMARY OF THE INVENTION

The present invention is set forth and characterized in the independent claims. The dependent claims describe other characteristics of the present invention or variants to the main inventive idea.

The invention concerns a positioning and support device provided with a containing chamber suitable to contain at least the distal end of an endoscopic instrument, for example the terminal part provided with an elevator to be subjected to washing treatment, and possible sterilization and/or disinfection.

The positioning and support device can be used on its own or in association with machines that carry out the treatment of endoscopic instruments, for example cooperating with baskets or trays of known treatment and washing machines.

The positioning and support device according to the invention, while being advantageously suitable to house and subject to treatment the distal end of an endoscopic instrument, can also be used to support a terminal part of other articles, possibly of different shapes and types, for which it is necessary to carry out a targeted treatment.

According to the present invention, the positioning and support device comprises:
a first internal tubular element, defining a treatment chamber suitable to contain and house a distal end of an oblong body having a first entry aperture, configured to allow the insertion and removal of the distal end to subject to washing and a second exit aperture, opposite the entry aperture;
a second external tubular element, coaxially associated with the internal tubular element, so as to define with the latter a hollow space;
an entry for a treatment fluid located in correspondence with one end of the hollow space, to which means to introduce a treatment fluid can be connected;
wherein the internal tubular element has a body on which a plurality of through holes open, configured to put the hollow space and the treatment chamber in fluidic communication.

According to the invention, therefore, the positioning and support device comprises a fluidic circuit defined by the association of the two tubular elements, which has an entry in correspondence with the entry for the fluid, and an exit in correspondence with the second discharge aperture.

During use, a pressurized fluid can be introduced into the circuit by means of introduction means through the entry of the fluid, reaching and distributing itself in the hollow space from which, passing through the holes located on the body of the internal tubular element, it flows in the form of jets inside the chamber. Once in the chamber, the fluid, together with the possible dirt removed from the endoscope, flows outside through the exit aperture.

The treatment fluid is introduced into the hollow space in an axial direction, promoting its distribution in a circumferential direction.

Furthermore, according to the invention, the entry for the fluid and the discharge exit are made on a same side of the positioning and support device. Combined with the fact that the hollow space is at least partly closed on the opposite side, this allows on the one hand to guarantee that the fluid is distributed and is delivered with uniform pressure through all the through holes toward the device to be washed, and on the other to guarantee that no treatment fluid accumulates in the hollow space itself.

According to the invention, the positioning and support device comprises a compartment to distribute the fluid that has an annular shape, delimited on one side by an external wall in which the entry is made and, on the opposite side, by an internal wall comprising at least three through slits communicating with the hollow space. The distribution compartment downstream of the hollow space allows to obtain a uniform distribution of the flow in the latter, and therefore a better delivery of the washing liquid toward the instrument to be washed.

According to the invention, said external tubular element is provided with a separating wall, or partition, defining said internal wall and comprising said exit made in a central portion thereof and said through slits disposed equally distanced around said exit.

According to some embodiments, the through holes can be disposed substantially along the circumferential and longitudinal development of the treatment chamber.

The through holes are configured to generate a plurality of jets so as to act on the terminal end substantially at 360°.

According to some embodiments, the holes can be simple single-diameter holes, or they can be countersunk holes, for example having a smaller diameter toward the chamber so as to reduce the section for the exit of the treatment fluid and increase its pressure.

According to possible variants, the holes can have a helical development so as to generate rotating jets which act aggressively on said terminal part.

The holes can be evenly distributed on the periphery of the chamber, or they can be distributed differently according to the complexity of the zone on which the jets operate.

According to some embodiments, the holes can be arranged aligned on parallel lines.

According to other embodiments, the holes can be arranged according to a helix-shaped curve.

According to other embodiments, it can be provided that the holes have different sizes and/or orientation according to the position in which they are located inside the chamber.

According to some embodiments, the internal tubular element comprises an annular portion, protruding from an external surface, which haa a radial extension correlated to the width of the hollow space, and configured to act as closure of the latter's end when the internal cylinder is inserted in, and assembled to, the external cylinder.

According to some embodiments, the internal tubular element and the external tubular element are provided with respective and mating coupling members and joining members, suitable to guarantee a stable reciprocal coupling of the two elements.

According to other embodiments, the positioning and support device comprises a support base configured to support the external tubular element assembled to the internal tubular element so that the discharge aperture is positioned at a lower height with respect to the entry aperture.

Some embodiments described here also concern a method to carry out a washing, sterilization and/or disinfection treatment of an endoscopic instrument in a positioning and support device according to the invention.

The method provides to position the distal end of an oblong body in the treatment chamber, introduce a pressurized fluid into the hollow space so as to generate a plurality of sprays and jets of treatment fluid inside the treatment chamber directed toward different positions of the distal end, and discharge the treatment fluid together with possible residual dirt through the exit aperture.

According to some embodiments, it can be provided that the type and/or pressure of the treatment fluid are correlated to the type and/or conditions of the terminal part to be subjected to washing.

These and other aspects, characteristics and advantages of the present disclosure will be better understood with reference to the following description, drawings and attached claims. The drawings, which are integrated and form part of the present description, show some embodiments of the present invention, and together with the description, are intended to describe the principles of the disclosure.

The various aspects and characteristics described in the present description can be applied individually where possible. These individual aspects, for example aspects and characteristics described in the description or in the attached dependent claims, can be the object of divisional applications.

### ILLUSTRATION OF THE DRAWINGS

These and other characteristics of the invention will become apparent from the following description of some embodiments, given as a non-restrictive example with reference to the attached drawings wherein:
- fig. 1 is a front perspective view of a positioning and support device associated with a basket that can be used in a treatment machine;
- fig. 2 is a rear perspective view of a positioning and support device according to embodiments described here;
- fig. 3 is an exploded perspective view of a positioning and support device according to the invention,
- fig. 4 is a lateral section view of a positioning and support device according to the invention in which the paths of a treatment fluid are shown;
- fig. 4a is a detail of fig. 4;
- fig. 5 is a lateral section view of a positioning and support device according to the invention, which contains inside it a distal end of a duodenoscope;

To facilitate comprehension, the same reference numbers have been used, where possible, to identify identical common elements in the drawings. It is understood that elements and characteristics of one embodiment can conveniently be incorporated into other embodiments without further clarifications.

### DESCRIPTION OF EMBODIMENTS

We will now refer in detail to the various embodiments of the invention, of which one or more examples are shown in the attached drawings. Each example is supplied by way of illustration of the invention and shall not be understood as a limitation thereof. For example, the characteristics shown or described insomuch as they are part of one embodiment can be adopted on, or in association with, other embodiments to produce another embodiment. It is understood that the present invention shall include all such modifications and variants falling within the scope of the present claims.

Before describing these embodiments, we must also clarify that the present description is not limited in its application to details of the construction and disposition of the components as described in the following description using the attached drawings. The present description can provide other embodiments and can be obtained or executed in various other ways. We must also clarify that the phraseology and terminology used here is for the purposes of description only, and cannot be considered as limitative.

Embodiments described here concern a positioning and support device indicated as a whole with reference number 10 suitable to contain at least one distal end E of an oblong body, that is, having a prevalent size along a longitudinal development, for example the terminal part of an endoscopic instrument to be subjected to washing treatment, and possible sterilization and/or disinfection.

According to some embodiments, the positioning and support device 10 can be used on its own or in association with machines that carry out the treatment of endoscopic instruments.

According to the invention, the positioning and support device 10 comprises a first internal tubular element 11, which is internally hollow and defines a treatment chamber 12 configured to house and contain the distal end E of the article to be subjected to treatment, such as for example the terminal part of an endoscopic instrument.

The positioning and support device 10 also comprises an external tubular element 13, coaxially associated with the internal tubular element 11 so as to define a hollow space 14 with it.

The hollow space 14 can have an annular shape, with a substantially constant width along its longitudinal development, and is configured to act as a distribution chamber for a treatment fluid before its introduction into the chamber 12.

The internal tubular element 11 has a body 16 on which a plurality of through holes 18 open, configured to put the hollow space 14 in fluidic communication with the treatment chamber 12.

According to the invention the hollow space 14 is at least partially closed at one end and has, at the opposite end, an entry 15 for the treatment fluid suitable to be connected, during use, to means to feed the treatment fluid.

The hollow space 14 on the end opposite the entry 15 can be completely closed or partly closed, since one or more holes 18 for the passage of the treatment fluid can be provided.

The entry 15 for the fluid is configured to introduce the treatment fluid inside the hollow space 14 along an axial direction, that is, in a direction substantially parallel to a longitudinal axis of the positioning and support device 10.

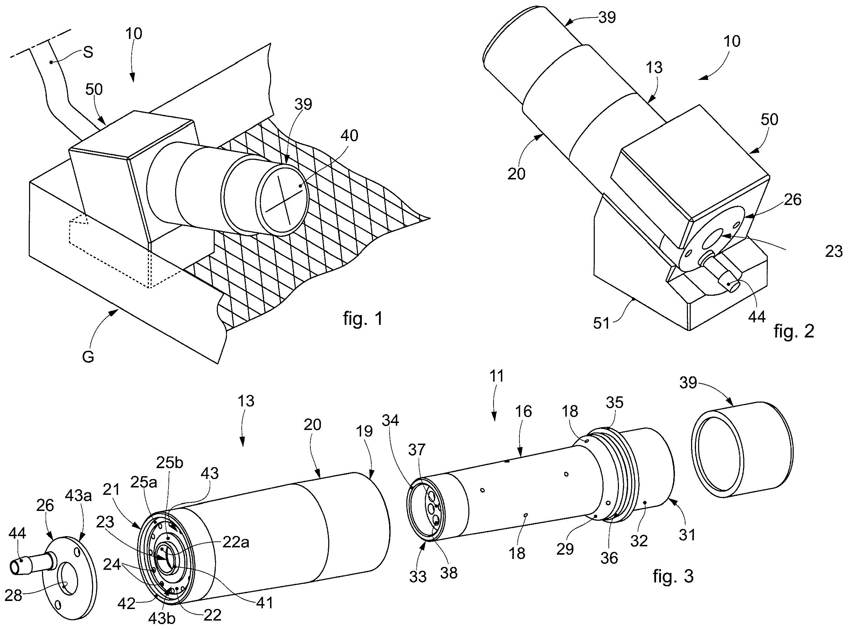

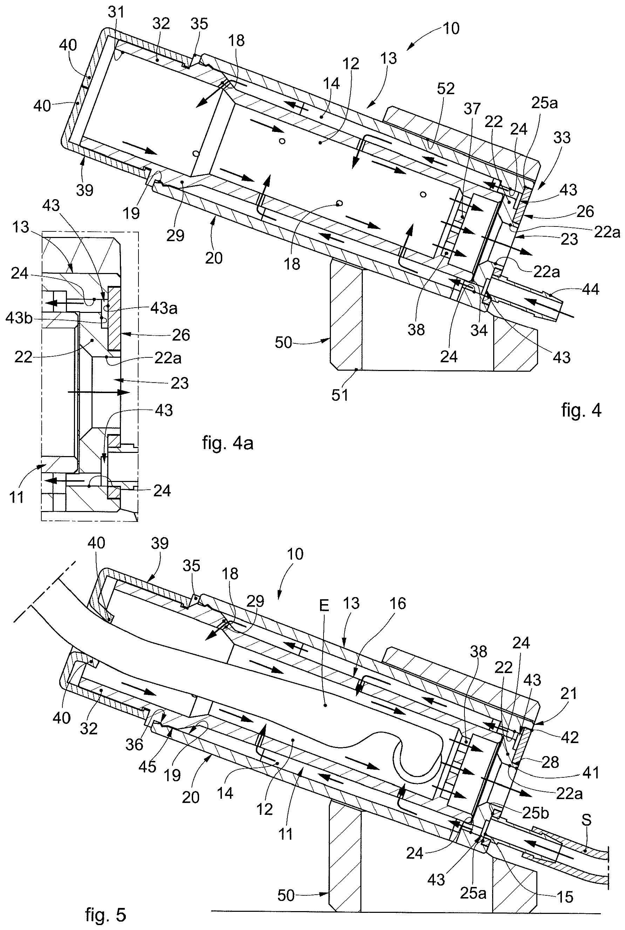

The positioning and support device 10 also includes an exit 23 communicating with the treatment chamber 12 and configured to allow the discharge and the outflow of the dirty fluid out of the chamber 12 (figs. 3 and 4).

The entry 15 for the fluid and the discharge exit 23 are made on a same side of the positioning and support device 10. In this way, possible residues of treatment fluid can also be advantageously discharged through the entry 15 without accumulating inside the hollow space 14.

According to some embodiments, for example described with reference to figs. 3 and 4, the external tubular element 13 has, in correspondence with a first end defining a neck portion 20, a first aperture 19, configured to allow the insertion therein of the internal tubular body 11.

The external tubular element 13 also comprises, in correspondence with a second end, opposite the first, and defining a bottom portion 21, the discharge exit 23. According to some embodiments, the external tubular element 13 can have a tapered external profile so that the aperture 19 has a slightly larger diameter than the bottom 21.

The aperture 19 can be sized to receive a suitable internal tubular element 11.

According to some embodiments, the elements 11, 13 can be made of polymeric plastic material, preferably polypropylene modified with the addition of glass fiber.

According to some embodiments, the elements 11, 13 can be made of the same material.

The use of polypropylene modified with the addition of glass fiber is particularly advantageous to withstand the conditions required in the washing and/or sterilization step. For example, it allows the device 10 to withstand high temperatures (over 100°C) and/or contact with acid or alkaline cleaning fluids and/or high pressures.

In other embodiments, the device 10 can be made of any other material whatsoever suited to the purposes of the invention.

The positioning and support device 10 between the entry 15 and the hollow space 14 comprises a compartment 43 to distribute the fluid which has an annular shape.

This distribution compartment is delimited on one side by an external wall 43a in which the entry 15 for the fluid is made and, on the opposite side, by an internal wall 43b comprising at least three through slits 24 communicating with the hollow space 14.

The washing fluid that is introduced through the entry 15 can then distribute itself evenly in the hollow space 14. The bottom portion 21 can have a separating wall, or partition 22, in the central portion of which there is made the at least one exit 23 for the outflow of the dirty treatment fluid coming from the treatment chamber 12.

The separating wall, or partition 22, defines the internal wall 43a of the distribution compartment 43.

The sizes of the exit 23 can be chosen based on the volume of fluid that has to be drained from the chamber 12 so that dirty fluid does not stagnate in the chamber 12.

According to some embodiments, the wall 22 is disposed in a recessed position with respect to an end edge of the external tubular body 13, that is, lying on a transverse plane that is more internal than that of the end of the bottom 21.

According to some embodiments, the exit 23 can have an annular edge 41 which extends longitudinally toward the bottom 21, protruding with respect to the external surface of the wall 22.

In accordance with some embodiments, the plane of the annular edge 41 of the exit 23 can substantially coincide with an edge 42 of the end of the bottom 21.

In some embodiments, on the circumferential end of the wall 22 there can be provided an abutment shoulder 25a that has a smaller extension than the edge 42 of the end of the bottom 21.

The wall 22 also comprises through slits 24 communicating on one side with the entry for the fluid and configured to allow the passage of the treatment fluid through them toward the hollow space 14.

According to some embodiments, there can be a plurality of slits 24 disposed along its circumferential development, and in communication with the hollow space 14.

According to the invention, at least three through slits 24 are provided, for example a number comprised between 3 and 15, as a function of their sizes and those of the external tubular element 13.

According to some embodiments, the slits 24 are disposed and shaped to provide a homogeneous flow of the fluid within the hollow space 14.

According to some embodiments, the slits 24 are disposed between the exit 23 and the abutment shoulder 25a.

According to the invention the slits 24 are disposed equally distanced around the exit 23.

In some embodiments, a second abutment shoulder 25b can be present between the slits 24 and the annular edge 41 of the exit 23.

According to some embodiments, the second abutment shoulder 25b has a plane substantially coinciding with the plane of the abutment edge 25a.

According to some embodiments, between the two abutment shoulders 25a and 25b there is defined the compartment 43 configured to receive the treatment fluid from the entry 15 for the fluid and distribute it toward the slits 24.

In accordance with some embodiments, the positioning and support device 10 comprises a closing device 26, configured to cooperate with the external tubular element 13 to delimit the compartment 43 with it.

In particular, the closing device 26 defines the external wall 43b of the compartment 43.

The closing device 26 can be ring-shaped, and be configured to rest on and be associated with the abutment shoulders 25a, 25b, so as to close the compartment 43 and seal it hermetically.

The closing device 26 comprises at least one aperture defining the entry for the fluid 15, communicating, during use, with the compartment 43 and the slits 24.

The closing device 26 can be provided with a central hole 28 configured to cooperate with the annular edge 41 of the exit 23, so as to guarantee its correct positioning.

In this way, advantageously, the exiting dirty fluid is kept separate from the entering clean fluid.

According to some embodiments, the closing device 26 can be made of a material different to that used for the tubular elements 11, 13, for example a metal alloy.

According to some embodiments, the closing device 26 can be attached to the bottom 21 by means of attachment means which can be, for example, screws, rivets, glues, adhesives or other.

According to some embodiments, the closing device 26 comprises a connector 44, fluidically communicating to the entry 15, and configured to be connected to feed means, or ducts S, suitable to deliver a fluid suitable to perform the tasks for which the device 10 is provided.

In accordance with some embodiments, the closing device 26 can be associated with the bottom 21 with attachment elements of the removable type so as to allow it to be replaced with one which, for example, provides a different connector 44 for association with different feed channels S.

Advantageously, the closing device 26 can also be removed in order to perform cleaning operations, for example, of slits 24, possibly and at least partly blocked.

According to some embodiments, the external tubular body 13 can comprise, in correspondence with the neck portion 20, coupling members 45 configured to cooperate with the internal tubular body 11 to allow a stable reciprocal coupling between the two elements.

According to some embodiments, the internal tubular element 11 comprises, in correspondence with a first end defining a neck portion 32, a first entry aperture 31 configured to allow the insertion in and removal from the treatment chamber 12 of the terminal end E to be washed.

The internal tubular element 11 also comprises, in correspondence with a second end, opposite the first, and defining a bottom portion 33, a separating wall or partition 37, provided with at least one through hole 38 through which the fluid of treatment can exit from chamber 12.

According to some embodiments, the internal tubular element 11 can have a body 16 having a tapered profile between the neck portion 32 and the bottom portion 33, such that the smaller diameter is in correspondence with the bottom 33.

According to some embodiments, the body 16 can have a variable thickness along its longitudinal and/or circumferential development.

According to some embodiments, the neck portion 32 can have a larger diameter than the body 16.

According to some embodiments, the through holes 18 can be made in the body 16 along its longitudinal extension between the neck portion 32 and the bottom portion 33.

According to some embodiments, the neck portion 32 and the bottom portion 33 can be substantially free of through holes 18.

According to some embodiments, the internal tubular element 11 can comprise a shoulder 29 which extends in a transverse direction with respect to the longitudinal axis and which has a larger diameter than that of the body 16.

According to some embodiments, the shoulder 29 has a diameter substantially equal to the internal diameter of the external tubular element 13, so that, during use, it is suitable to be positioned in contact with the latter in such a way as to close the hollow space 14 in correspondence with the neck portion 20 of the latter.

According to some embodiments, through holes 18 can be provided made in the thickness of the shoulder 29, suitable to allow the passage of the treatment fluid from the hollow space 14 toward the neck portion 32.

According to some embodiments, at the base of the neck 32 there can be a ring-shaped abutment portion 35, which protrudes beyond the shoulder 29, which is configured to position itself resting during use, on the upper edge of the first aperture 19 of the external tubular element 13 when the internal tubular element 11 is inserted into it.

According to other embodiments, the internal tubular element 11 can have joining members 36 suitable to couple with the respective coupling members 45 of the external tubular element 13 so as to generate a substantially hermetic coupling.

In accordance with some embodiments, the coupling of the joining members 36 with the respective coupling members 45, can be either same shape, or snap-in, or by screwing or other suitable types.

By way of example, the joining members 36 can comprise a protruding tooth, while the coupling members 45 can comprise an attachment seating suitable to receive the tooth, thereby producing a same shape snap-in coupling with it.

In accordance with some embodiments, the partition 37 can be positioned on a more internal plane than the plane of the end of the bottom 33.

According to some embodiments, between the separating wall, or partition 37 and the end of the bottom portion 33, there extends a ring-shaped portion 34 that is full, that is, without holes, which, during use, positions itself resting against the internal surface of the separating wall 22 of the external tubular element 13.

In this way, it is guaranteed that the clean treatment fluid introduced into the hollow space 14 is not in any way contaminated with the dirty treatment fluid exiting the treatment chamber 12.

According to some embodiments, the body 16 defines the circular perimeter of the chamber 12 which develops substantially from the separating wall, or partition 37, up to, ideally, in correspondence with the shoulder 29.

The sizes of the chamber 12 can be correlated at least to the type of distal end E of the endoscope to be subjected to treatment.

According to some embodiments, the separating wall, or partition 37 can be advantageously provided to prevent the distal end E of the instrument to be cleaned from exiting from the chamber 12, nullifying the cleaning and/or possibly sterilization treatment.

According to some embodiments, the separating wall, or partition 37 comprises a plurality of slits, or through holes 38, for example arranged along one or more circumferences.

According to some embodiments, the slits 38 or holes can have a size sufficient to allow possible residues of dirt to exit from the chamber 12 preventing clogging.

According to some embodiments, the hollow space 14 and the chamber 12 are in fluidic communication by means of holes 18 provided on the body 16.

The holes 18 can be simple single-diameter holes, or countersunk closing advantageously toward the chamber 12 to generate a greater pressure.

In other embodiments, the holes 18 can have different sizes.

The disposition of the holes 18 on the chamber 12 can be provided, for example in the shape of a helix, so as to generate rotating jets.

The holes 18 can be equally distributed or be disposed with a higher density in correspondence with and/or according to the complexity of the zone on which the jets operate.

In accordance with some embodiments, the holes 18 can have a disposition pattern according to the conformation of the distal end E of the endoscope to be cleaned.

According to this variant, indication elements can be provided, not shown, suitable to indicate the correct insertion orientation of the distal end E.

According to some embodiments, the positioning and support device 10 also comprises a closing element 39, for example a lid or a cap, configured to at least partly close the entry aperture 31 to the chamber 12.

In accordance with some embodiments, the closing device 26 can be provided to associate with the neck portion 32. For example, the closing element 39 can have a cylindrical shape, with a diameter suitable to couple by interference with the neck portion 32.

According to some embodiments, the closing element 39 can position itself, during use, with a bottom wall 46 thereof abutting against the edge of the entry aperture 31 and with the edge of the end open against the abutment portion 35.

According to some embodiments, the closing element 39 can be made of elastomeric material, preferably silicone or other material suitable to perform the function for which it is provided.

The element 39 can be provided, in its own bottom wall 46, with a cut, for example cross-shaped, which generates a plurality of flaps 40 configured to receive the endoscope by flexing toward the inside of the device 10 simultaneously with the introduction of the distal end E of the endoscope.

Advantageously, the flaps 40 allow to maintain the distal end E of the endoscope substantially centered inside the chamber 12 and they limit, during use, the exit of treatment fluid from the latter.

According to some embodiments, the positioning and support device 10 also comprises a support base 50 suitable to support and position the assembly consisting of the external tubular element 13 and the internal tubular element 11 inserted in it.

According to some embodiments, the support base 50 comprises a support surface 51 suitable to be positioned, during use, resting on a work surface, or on a basket of a treatment machine, and a housing seating 52 configured to partly house the external tubular element 13.

The housing seating 52 has a central axis C inclined with respect to the support surface 51, so as to position the assembly consisting of the external tubular element 13 and the internal tubular element 11 with the inclined longitudinal axis, promoting the outflow of the treatment fluid toward the discharge exit 23.

By way of example, the central axis C can have an angle of inclination α comprised between about 10° and about 20°.

According to some embodiments, the housing seating 52 has a tapered shape, with a shape and sizes correlated to the tapered shape of the external tubular element 13, so as to obtain with it a coupling by interference, clamping it in the desired position.

Advantageously, the external profile of the external tubular element 13 can be suitably conformed to couple through a same shape coupling with the support base 50.

The support base 50 can be provided with attachment elements, such as, for example, non-dryable glues, screws, or other, in order to be removably attached to a basket G, or a tray of a treatment machine (fig. 1).

The invention also concerns a method according to claim 11, at to wash a distal end E of an endoscopic instrument with a positioning and support device 10 according to the invention.

The washing method comprises:
- positioning the distal end E to be subjected to treatment in chamber 12;
- connecting the entry for fluid 15 to means to feed a treatment fluid;
- introducing a treatment fluid through the entry 15 for the fluid so that it is distributed in the hollow space 14, passes through the through holes 18 and is delivered in the form of jets into the treatment chamber 12, acting on the distal end E from different directions;
- discharging the treatment fluid with the possible residual dirt from the treatment chamber 12 through the discharge exit 23.

In particular, the method provides to introduce the fluid in the hollow space 14 in an axial direction, from the same side of the discharge exit 23.

According to some embodiments, the method provides to position the assembly consisting of the external tubular element 13 and the internal tubular element 11 on a support base 50 so as to position them with their longitudinal axis inclined with respect to the horizontal, with the discharge exit 23 positioned lower than the entry aperture 31.

According to some embodiments, the method provides to deliver a pressurized treatment fluid, the delivery pressure of which can be correlated to the shape, type, or even the condition of maximum contamination of the distal end E of the endoscopic instrument to be cleaned.

According to the invention the treatment fluid is introduced inside the annular-shaped distribution compartment 43 located upstream of the hollow space 14, it is distributed in the latter and is introduced uniformly into the hollow space 14 through the plurality of through slits 24.

It is clear that modifications and/or additions of parts may be made to the positioning and support device 10 and to the washing method as described heretofore, without departing from the field and scope of the present invention, as defined in the claims.

It is also clear that, although the present invention has been described with reference to some specific examples, a person of skill in the art shall certainly be able to achieve many other equivalent forms of positioning and support device 10 and washing method, having the characteristics as set forth in the claims and hence all coming within the field of protection defined thereby.

In the following claims, the sole purpose of the references in brackets is to facilitate reading: they must not be considered as restrictive factors with regard to the field of protection claimed in the specific claims.

## Claims

1. Positioning and support device suitable to contain a distal end (E) of an oblong article to be subjected to washing treatment, and possible sterilization and/or disinfection, comprising:
a first internal tubular element (11) defining a treatment chamber (12) suitable to contain and house said distal end (E), and having an entry aperture (31), configured to allow the insertion in and removal from said chamber (12) of said distal end (E), wherein said internal tubular element (11) has a body (16) onto which a plurality of through holes (18) open;
a second external tubular element (13), coaxially associated with said internal tubular element (11), so as to define with the latter a hollow space (14) fluidically connected to said treatment chamber (12) by means of said through holes (18);
an entry (15) for a treatment fluid located in correspondence with a first end (10a) of said positioning and support device, to which means (44, S) to introduce a treatment fluid can be connected, and oriented in an axial direction with respect to said hollow space (14) and
a discharge exit (23) configured to allow the outflow of the treatment fluid from said chamber (12), **characterized in that**
said entry (15) and said discharge exit (23) are disposed on a same end of said positioning and support device and said hollow space (14) is at least partly closed in correspondence with the opposite end,
said positioning and support device comprises a compartment (43) to distribute the fluid that has an annular shape, delimited on one side by an external wall (43a) in which said entry (15) is made and, on the opposite side, by an internal wall (43b) comprising at least three through slits (24) communicating with said hollow space (14) and
said external tubular element (13) is provided with a separating wall, or partition (22), defining said internal wall (43b) and comprising said exit (23) made in a central portion thereof and said through slits (24) disposed equally distanced around said exit (23).

2. Positioning and support device as in claim 1, **characterized in that** there is provided a number of through slits (24) comprised between 3 and 15.

3. Positioning and support device as in claim 1 or 2, **characterized in that** said separating wall, or partition (22), comprises a first abutment shoulder (25a) located externally to said slits (24) and a second abutment shoulder (25b) located between said exit (23) and said slits (24), having a longitudinal extension below the edge (42) of the bottom end (21) of said external tubular element (13) and defining said distribution compartment (43).

4. Positioning and support device as in any previous claim from 1 to 3, **characterized in that** it comprises a closing device (26), defining said external wall (43a) and configured to cooperate with said external tubular element (13) to close said distribution compartment (43) and said hollow space (14), said closing device (26) being provided with at least one aperture defining the entry for the fluid (15) and a connector (44) associated with said aperture.

5. Positioning and support device as in any claim hereinbefore, **characterized in that** said through holes (18) are disposed substantially along the circumferential and longitudinal development of said treatment chamber (12) arranged according to a defined pattern.

6. Positioning and support device as in any claim hereinbefore, **characterized in that** said external tubular element (13) comprises a neck portion (20), in correspondence with which a first aperture (19) is provided, configured to allow the insertion of said internal tubular body (11) and a bottom portion (21) in correspondence with which said discharge exit (23) is provided.

7. Positioning and support device as in any claim hereinbefore, **characterized in that** said internal tubular element (11) comprises a neck portion (32), in correspondence with which said entry aperture (31) is provided, and a bottom portion (33) in correspondence with which at least one through hole (38) is provided, configured to allow the exit of the treatment fluid from said chamber (12).

8. Positioning and support device as in any claim hereinbefore, **characterized in that** said internal tubular element (11) and said external tubular element (13) comprise respective and mating coupling members (45) and joining members (36) configured to allow a stable reciprocal coupling between them.

9. Positioning and support device as in any claim hereinbefore, **characterized in that** it comprises a support base (50) configured to support the assembly consisting of said external tubular element (13) and said internal tubular element (11) so that said discharge aperture (23) is positioned at a lower height with respect to said entry aperture (31).

10. Positioning and support device as in any claim hereinbefore, **characterized in that** it comprises a closing element (39), configured to cooperate with said internal tubular element (11) in order to at least partially close said entry aperture (31), in any case allowing said distal end (E) to transit through it.

11. Method to subject to washing a distal end (E) of an endoscopic instrument with a positioning and support device (10) as in any claim hereinbefore, **characterized in that** it comprises:
- positioning said distal end (E) to be subjected to treatment in said chamber (12);
- connecting said entry (15) for the fluid to means to feed a treatment fluid;
- introducing a treatment fluid through said entry (15) for the fluid in an axial direction into the annular-shaped distribution compartment (43), so that the fluid is distributed in the distribution compartment (43), is introduced in said hollow space (14) through the through slits (24), is distributed in the hollow space (14), transits through the through holes (18) and is delivered in the form of jets in the treatment chamber (12), acting on the distal end (E) from different directions;
- discharging the treatment fluid with the possible residual dirt from the treatment chamber (12) through the discharge exit (23).

12. Method as in claim 11, **characterized in that** it provides to position said exit aperture (23) at a lower height than said entry aperture (31).

## Patentansprüche

1. Positionier- und Stützvorrichtung, die geeignet ist, ein distales Ende (E) eines länglichen Gegenstands aufzunehmen, der einer Waschbehandlung und möglicherweise einer Sterilisation und/oder Desinfektion unterzogen werden soll, umfassend: ein erstes inneres rohrförmiges Element (11), das eine Kammer (12) definiert, die geeignet ist, das distale Ende (E) zu enthalten und aufzunehmen, und das eine Eintrittsöffnung (31) aufweist, die so ausgebildet ist, dass sie das Einführen des distalen Endes (E) in die Kammer (12) und das Entfernen des distalen Endes (E) aus der Kammer (12) ermöglicht, wobei das innere rohrförmige Element (11) einen Körper (16) aufweist, in den eine Mehrzahl von Durchgangslöchern (18) mündet; ein zweites äußeres rohrförmiges Element (13), das koaxial mit dem inneren rohrförmigen Element (11) verbunden ist, um mit diesem einen Hohlraum (14) zu bilden, der über die Durchgangslöcher (18) mit der Kammer (12) fluidverbunden ist; einen Eingang (15) für ein Behandlungsfluid, der in Übereinstimmung mit einem ersten Ende (10a) der Positionierungs- und Stützvorrichtung angeordnet ist, an den Mittel (44, S) zum Einleiten eines Behandlungsfluids angeschlossen werden können und der in einer axialen Richtung in Bezug auf den Hohlraum (14) ausgerichtet ist, und einen Ausgang (23), der so ausgebildet ist, dass er das Ausströmen des Behandlungsfluids aus der Kammer (12) ermöglicht, **dadurch gekennzeichnet, dass** der Eingang (15) und der Ausgang (23) an demselben Ende der Positionier- und Stützvorrichtung angeordnet sind und der Hohlraum (14) zumindest teilweise dem gegenüberliegenden Ende geschlossen ist, die Positionierungs- und Stützvorrichtung einen Verteilungskanal (43) zur Verteilung des Fluids umfasst, der eine ringförmige Form aufweist, die auf einer Seite durch eine Außenwand (43a), in der der Eingang (15) erfolgt, und auf der gegenüberliegenden Seite durch eine Innenwand (43b) begrenzt ist, die mindestens drei Durchgangsschlitze (24) aufweist, die mit dem Hohlraum (14) in Verbindung stehen, und das äußere rohrförmige Element (13) mit einer Trennwand oder Zwischenwand (22) versehen ist, die die Innenwand (43b) definiert und den Ausgang (23) aufweist, der in einem zentralen Abschnitt davon ausgebildet ist, und in dem gleichen Abstand um den Ausgang (23) herum angeordneten Durchgangsschlitze (24) umfasst.

2. Positionier- und Stützvorrichtung nach Anspruch 1, **dadurch gekennzeichnet, dass** eine Anzahl von Durchgangsschlitzen (24) zwischen 3 und 15 vorgesehen ist.

3. Positionier- und Stützvorrichtung nach Anspruch 1 oder 2, **dadurch gekennzeichnet, dass** die Trennwand oder Teilung (22) eine erste Anlageschulter (25a), die sich außerhalb der Durchgangsschlitze (24) befindet, und eine zweite Anlageschulter (25b), die sich zwischen dem Ausgang (23) und den Durchgangsschlitzen (24) befindet, umfasst, die eine Längserstreckung unterhalb des Randes (42) des unteren Bodenabschnitts (21) des äußeren rohrförmigen Elements (13) aufweist und die den Verteilungskanal (43) definiert.

4. Positionierungs- und Stützvorrichtung nach einem der vorhergehenden Ansprüche 1 bis 3, **dadurch gekennzeichnet, dass** sie eine Verschlussvorrichtung (26) umfasst, die die Außenwand (43a) definiert und so konfiguriert ist, dass sie mit dem äußeren rohrförmigen Element (13) zusammenwirkt, um den Verteilungskanal (43) und den Hohlraum (14) zu verschließen, wobei die Verschlussvorrichtung (26) mit mindestens einer Öffnung, die den Eingang für das Fluid (15) definiert, und einem mit der Öffnung verbundenen Anschlussstück (44) versehen ist.

5. Positionier- und Stützvorrichtung nach einem der vorhergehenden Ansprüche, **dadurch gekennzeichnet, dass** die Durchgangslöcher (18) im Wesentlichen entlang der Umfangs- und Längserstreckung der Kammer (12) nach einem definierten Muster angeordnet sind.

6. Positionier- und Stützvorrichtung nach einem der vorhergehenden Ansprüche, **dadurch gekennzeichnet, dass** das äußere rohrförmige Element (13) einen Halsabschnitt (20), in dem eine erste Öffnung (19) angeordnet ist, die so ausgebildet ist, dass sie das Einführen des inneren rohrförmigen Körpers (11) ermöglicht, und einen Bodenabschnitt (21) umfasst, in dem der Ausgang (23) angeordnet ist.

7. Positionier- und Stützvorrichtung nach einem der vorhergehenden Ansprüche, **dadurch gekennzeichnet, dass** das innere rohrförmige Element (11) einen Halsabschnitt (32), in dem die Eintrittsöffnung (31) angeordnet ist, und einen Bodenabschnitt (33) umfasst, in dem mindestens ein Durchgangsloch (38) angeordnet ist, das so ausgebildet ist, dass es den Austritt des Behandlungsfluids aus der Kammer (12) ermöglicht.

8. Positionier- und Stützvorrichtung nach einem der vorhergehenden Ansprüche, **dadurch gekennzeichnet, dass** das innere rohrförmige Element (11) und das äußere rohrförmige Element (13) entsprechende und zusammenpassende Kopplungselemente (45) und Verbindungselemente (36) umfassen, die so ausgebildet sind, dass sie eine stabile gegenseitige Kopplung zwischen ihnen ermöglichen.

9. Positionier- und Stützvorrichtung nach einem der vorhergehenden Ansprüche, **dadurch gekennzeichnet, dass** sie eine Stützbasis (50) umfasst, die so ausgebildet ist, dass sie die aus dem äußeren rohrförmigen Element (13) und dem inneren rohrförmigen Element (11) bestehende Anordnung stützt, so dass die Auslassöffnung (23) in geringerer Höhe gegenüber der Eintrittsöffnung (31) angeordnet ist.

10. Positionier- und Stützvorrichtung nach einem der vorhergehenden Ansprüche, **dadurch gekennzeichnet, dass** sie ein Verschlusselement (39) umfasst, das so ausgebildet ist, dass es mit dem inneren rohrförmigen Element (11) zusammenwirkt, um die Eintrittsöffnung (31) zumindest teilweise zu verschließen, wobei in jedem Fall der Durchgang des distalen Endes (E) ermöglicht wird.

11. Verfahren zum Waschen eines distalen Endes (E) eines endoskopischen Instruments mit einer Positionier- und Stützvorrichtung (10) nach einem der vorhergehenden Ansprüche, **dadurch gekennzeichnet, dass** es Folgendes umfasst:
- Positionieren des distalen Endes (E), das der Behandlung unterzogen werden soll, in der Kammer (12);
- Verbinden des Eintritts (15) für das Fluid mit Mitteln zur Zufuhr eines Behandlungsfluids;
- Einleiten eines Behandlungsfluids durch den Eingang (15) für das Fluid in einer axialen Richtung in den ringförmigen Verteilungskanal (43), so dass das Fluid in dem Verteilungskanal (43) verteilt wird, in den Hohlraum (14) durch die Durchgangsschlitze (24) eingeleitet wird, in dem Hohlraum (14) verteilt wird, durch die Durchgangslöcher (18) hindurchtritt und in Form von Strahlen in die Kammer (12) abgegeben wird, die aus verschiedenen Richtungen auf das distale Ende (E) wirken;
- Ableiten der Behandlungsflüssigkeit mit dem eventuellen Restschmutz aus der Kammer (12) durch den Ausgang (23).

12. Verfahren nach Anspruch 11, **dadurch gekennzeichnet, dass** es vorsieht, den Ausgang (23) niedriger als die Eingangsöffnung (31) anzuordnen.

## Revendications

1. Dispositif de positionnement et de support approprié pour contenir une extrémité distale (E) d'un article oblong devant être soumis à un traitement de lavage, et facultativement à une stérilisation et/ou une désinfection, comprenant :
un premier élément tubulaire interne (11) définissant une chambre de traitement (12) appropriée pour contenir et loger ladite extrémité distale (E), et présentant une ouverture d'entrée (31), configurée pour permettre l'insertion dans et le retrait à partir de ladite chambre (12) de ladite extrémité distale (E), dans lequel ledit élément tubulaire interne (11) présente un corps (16) sur lequel une pluralité de trous traversants (18) s'ouvrent ;
un second élément tubulaire externe (13), associé coaxialement audit élément tubulaire interne (11), de manière à définir avec ce dernier un espace creux (14) relié de manière fluidique à ladite chambre de traitement (12) au moyen desdits trous traversants (18) ;
une entrée (15) pour un fluide de traitement située en correspondance avec une première extrémité (10a) dudit dispositif de positionnement et de support, à laquelle des moyens (44, S) pour introduire un fluide de traitement peuvent être reliés, et orientée dans une direction axiale par rapport audit espace creux (14), et
une sortie de décharge (23) configurée pour permettre l'écoulement sortant du fluide de traitement à partir de ladite chambre (12), **caractérisé en ce que**
ladite entrée (15) et ladite sortie de décharge (23) sont disposées sur une même extrémité dudit dispositif de positionnement et de support et ledit espace creux (14) est au moins partiellement fermé en correspondance avec l'extrémité opposée,
ledit dispositif de positionnement et de support comprend un compartiment (43) de distribution du fluide qui présente une forme annulaire, délimité d'un côté par une paroi externe (43a) dans laquelle est ménagée ladite entrée (15) et, du côté opposé, par une paroi interne (43b) comprenant au moins trois fentes traversantes (24) communiquant avec ledit espace creux (14) et
ledit élément tubulaire externe (13) est pourvu d'une paroi de séparation, ou cloison (22), définissant ladite paroi interne (43b)
et comprenant ladite sortie (23) réalisée dans une partie centrale de celle-ci et lesdites fentes traversantes (24) disposées à égale distance autour de ladite sortie (23).

2. Dispositif de positionnement et de support selon la revendication 1, **caractérisé en ce qu'**un nombre de fentes traversantes (24) est prévu, compris entre 3 et 15.

3. Dispositif de positionnement et de support selon la revendication 1 ou 2, **caractérisé en ce que** ladite paroi de séparation, ou cloison (22), comprend un premier épaulement de butée (25a) situé à l'extérieur desdites fentes (24) et un second épaulement de butée (25b) situé entre ladite sortie (23) et lesdites fentes (24), présentant une extension longitudinale en dessous du bord (42) de l'extrémité inférieure (21) dudit élément tubulaire externe (13) et définissant ledit compartiment de distribution (43).

4. Dispositif de positionnement et de support selon l'une quelconque des revendications précédentes de 1 à 3, **caractérisé en ce qu'**il comprend un dispositif de fermeture (26), définissant ladite paroi externe (43a) et configuré pour coopérer avec ledit élément tubulaire externe (13) pour fermer ledit compartiment de distribution (43) et ledit espace creux (14), ledit dispositif de fermeture (26) étant pourvu d'au moins une ouverture définissant l'entrée pour le fluide (15) et d'un connecteur (44) associé à ladite ouverture.

5. Dispositif de positionnement et de support selon l'une quelconque des revendications précédentes, **caractérisé en ce que** lesdits trous traversants (18) sont disposés sensiblement le long du développement circonférentiel et longitudinal de ladite chambre de traitement (12) agencée selon un motif défini.

6. Dispositif de positionnement et de support selon l'une quelconque des revendications précédentes, **caractérisé en ce que** ledit élément tubulaire externe (13) comprend une partie de col (20), avec laquelle une première ouverture (19) est prévue en correspondance, configurée pour permettre l'insertion dudit corps tubulaire interne (11), et une partie de fond (21) avec laquelle ladite sortie de décharge (23) est prévue en correspondance.

7. Dispositif de positionnement et de support selon l'une quelconque des revendications précédentes, **caractérisé en ce que** ledit élément tubulaire interne (11) comprend une partie de col (32), avec laquelle ladite ouverture d'entrée (31) est prévue en correspondance, et une partie de fond (33) avec laquelle au moins un trou traversant (38) est prévu en correspondance, configurée pour permettre la sortie du fluide de traitement à partir de ladite chambre (12).

8. Dispositif de positionnement et de support selon l'une quelconque des revendications précédentes, **caractérisé en ce que** ledit élément tubulaire interne (11) et ledit élément tubulaire externe (13) comprennent des éléments de couplage respectifs et d'appariement (45) et des éléments de jonction (36) configurés pour permettre un couplage réciproque stable entre eux.

9. Dispositif de positionnement et de support selon l'une quelconque des revendications précédentes, **caractérisé en ce qu'**il comprend une base de support (50) configurée pour supporter l'ensemble constitué dudit élément tubulaire externe (13) et dudit élément tubulaire interne (11) de sorte que ladite ouverture de décharge (23) est positionnée à une hauteur inférieure par rapport à ladite ouverture d'entrée (31).

10. Dispositif de positionnement et de support selon l'une quelconque des revendications précédentes, **caractérisé en ce qu'**il comprend un élément de fermeture (39), configuré pour coopérer avec ledit élément tubulaire interne (11) afin de fermer au moins partiellement ladite ouverture d'entrée (31), dans tous les cas permettant à ladite extrémité distale (E) de la traverser.

11. Procédé pour soumettre à un lavage une extrémité distale (E) d'un instrument endoscopique avec un dispositif de positionnement et de support (10) selon l'une quelconque des revendications précédentes, **caractérisé en ce qu'**il comprend les étapes consistant à :
- positionner ladite extrémité distale (E) devant être soumise à un traitement dans ladite chambre (12) ;
- relier ladite entrée (15) du fluide à des moyens d'alimentation en fluide de traitement ;
- introduire un fluide de traitement à travers ladite entrée (15) du fluide dans une direction axiale dans le compartiment de distribution de forme annulaire (43), de sorte que le fluide est distribué dans le compartiment de distribution (43), est introduit dans ledit espace creux (14) à travers les fentes traversantes (24), est distribué dans l'espace creux (14), traverse les trous traversants (18) et est délivré sous la forme de jets dans la chambre de traitement (12), en agissant sur l'extrémité distale (E) à partir de différentes directions ;
- décharger le fluide de traitement avec les éventuelles saletés résiduelles à partir de la chambre de traitement (12) à travers la sortie de décharge (23).

12. Procédé selon la revendication 11, **caractérisé en ce qu'**il prévoit de positionner ladite ouverture de sortie (23) à une hauteur inférieure à celle de ladite ouverture d'entrée (31).
